Europäisches Patentamt

(19) European Patent Office

Office européen des brevets

(11) Publication number: **0 167 300**
A1

(12) **EUROPEAN PATENT APPLICATION**

(21) Application number: **85304006.1**

(22) Date of filing: **05.06.85**

(51) Int. Cl.⁴: **C 07 C 29/136**
C 07 C 29/15, C 07 C 31/00

(30) Priority: 06.06.84 GB 8414473
18.06.84 GB 8415458
20.06.84 GB 8415661
28.06.84 GB 8416470
28.06.84 GB 8416471
04.12.84 GB 8430515

(43) Date of publication of application:
08.01.86 Bulletin 86/2

(84) Designated Contracting States:
DE FR GB IT

(71) Applicant: Humphreys & Glasgow Limited
Chestergate House 253 Vauxhall Bridge Road
GB-London SW1V 1HD(GB)

(72) Inventor: Winter, Christopher Leslie
1 Burrows Close
Great Bookham Surrey, KY23 3HB(GB)

(74) Representative: Wise, Stephen James et al,
c/o RAWORTH, MOSS & COOK 36 Sydenham Road
Croydon, Surrey CR0 2EF(GB)

(54) **Process for the production of alcohols.**

㉗ A process is disclosed for the production of an aliphatic alcohol having at least two carbon atoms, preferably ethanol, from a carbonaceous feedstock, preferably natural gas, via an intermediate aliphatic alcohol having one less carbon atom, preferably methanol, via an intermediate compound containing the group $CH_3(CH_2)n\ C(O)-$, preferably acetic acid.

The feedstock is reformed and the synthesis gas formed is separated, preferably by a PSA unit, into three different streams which are used in the three stage process, one of which streams is a pure hydrogen stream which can be used for the concurrent production of ammonia.

The ethanol formed is useful as a petrol extender and octane improver for automobile fuel.

EP 0 167 300 A1

PROCESS FOR THE PRODUCTION OF ALCOHOLS

The present invention relates to a process for producing an aliphatic alcohol, preferably ethanol, particularly in concert with ammonia. Although the present invention will be described with particular reference to the use of natural gas as the starting material, hydrocarbons other than natural gas, and carbonaceous materials in general, can also be utilized as the source of carbon for the process of this invention.

There are many places in the world where natural gas is available very cheaply. Indeed sometimes it is freely available because it can be a by-product of oil or LPG production, and many countries still flare-off substantial quantities of the gas.

Until recently the main outlets for natural gas have been methanol, ammonia and LNG. Currently, there is a substantial excess of methanol capacity, and some LNG projects have become stalled because of their very large capital cost, the need for dedicated facilities at the receiving end, and dedicated supply/demand problems. Ammonia is really only a possibility where there is a ready market for finished fertilizer which constitutes its main product. Hydrocarbon and "syncrude" production processes are also now appearing commercially, but the end products are relatively expensive.

Recently, considerable concern has arisen about the use of lead compounds in the petrol used in cars, but so far lead substitutes have not been economic. For example, MTBE (Methyl Tertiary Butyl Ether) has not been economically available in sufficient quantities, and the use of methanol alone has not been well received because of gasket problems, the adverse publicity caused by phase separation which can occur when petrol-containing methanol is in contact with water, and leaning-out problems.

Of the lower aliphatic alcohols, ethanol is a proven petrol extender and octane improver, and its use in petrol helps to reduce or even eliminate the need for lead antiknock compounds. Petrol formulations incorporating ethanol are well known to those skilled in the art. Although the present invention will be described with particular reference to the production of ethanol, it will be appreciated that the general process can be used to produce higher aliphatic alcohols, e.g. propanol.

In accordance with the present invention there is provided a process for the production of an aliphatic alcohol having at least two carbon atoms from a carbonaceous feedstock, which process comprises:

a) reforming the feedstock to yield synthesis gas containing carbon monoxide, optionally together with carbon dioxide, and hydrogen,

b) reacting together some of the carbon oxide(s) from step (a) and some of the hydrogen from step (a) to form an intermediate aliphatic alcohol having at least one carbon atom,

c) reacting together carbon monoxide from step (a) and the intermediate alcohol from step (b) to form an intermediate compound containing the group $CH_3(CH_2)n\ C(O)-$ wherein n is 0 or a positive integer, and

d) reacting the intermediate compound from step (c) with more of the hydrogen from step (a) to form the desired aliphatic alcohol,

wherein step (a) includes the separation of at least some of the synthesis gas whereby step (a) produces:-

A. a stream containing hydrogen, a low (relative to Stream B. below) concentration of carbon monoxide and a balance of the other gases in the synthesis gas,

B. a stream rich in carbon monoxide, and

C. a stream containing substantially pure hydrogen,

wherein at least some of Stream A is reacted in step (b), at least some of Stream B is reacted in step (c), and at least some of Stream C is reacted in step (d).

Preferably the process of the invention utilizes natural gas to produce ethanol via methanol and a compound containing the group $CH_3C(O)-$, e.g. acetic acid, ethyl acetate or acetic anhydride, all of which compounds are sometimes referred to generally as "acetates". Most preferably the intermediate compound is the alkanoic acid, which for ethanol production is acetic acid.

In its preferred embodiment, the present process includes the surprising proposal to take acetic acid, which currently cost U.S.$550/tonne, spend money on processing it to ethanol, with a concommitant loss of weight (yield), sell the ethanol at about U.S.$300/tonne, and still make a profit.

Although several gas separation techniques, e.g. cryogenic separation, can be used in the production of Streams A, B and C, it is best, both economically and practically, to utilize a molecular sieve separation technique, and the use of this technique will be described hereinafter.

In a most preferred embodiment, ammonia is concurrently produced from excess hydrogen produced in the main ethanol-production process.

The first step of the preferred process comprises reforming natural gas to produce synthesis gas, i.e. gas containing hydrogen and least one oxide of carbon, usually predominantly carbon monoxide. Preferably this reforming would be undertaken in at least one tubular steam reformer, but at least one partial oxidation reformer using an oxygen-containing gas, preferably steam, or a combination of the two methods of reforming could also be used. This synthesis gas is then used, after a gas separation step, to produce methanol, which in turn is fed into a standard "acetate", preferably acetic acid, production process. The well-known acetic acid process developed by Monsanto is particularly preferred.

Unexpectedly, it has been found that acetic acid can be made by a conventional process using relatively low purity carbon monoxide, e.g. below 92 mol %, compared with the present practice of using about 98% CO, i.e. the process of the invention can use carbon monoxide which contains at least four times the currently acceptable impurity content. Indeed, there are both direct and indirect advantages of utilizing such an "impure" CO stream in a conventional liquid phase CO reactor.

The main direct advantage is that, given that the partial pressure of the carbon monoxide cannot be reduced for reasons of maintenance of the chemical equilibrium, the more impure the carbon monoxide stream the more unreacted gas passes through the reaction fluid in which the acetic acid or other acetate is produced using well known catalytic systems. Thus for a given gas sparging system, the bubbles of the carbon monoxide stream fed into the reactor are either more numerous or larger, or a combination of the two. Thus for a given gas-liquid contact time, i.e. reactor configuration, the area for carbon monoxide mass transfer from the gas to the liquid is larger, and thus more CO can be transferred into the liquid phase with "impure" CO than with "pure" CO.

The most important indirect advantage is that, for the gas separator step, molecular sieve adsorption techniques can be used to produce this lower purity carbon monoxide stream, whereas they are not economic for the production of the heretofore-used high purity carbon monoxide stream. This in turn leads to the further advantage of the concomitant production of very high purity hydrogen from which very cheap ammonia can be made.

If the natural gas feed contains nitrogen, as is the case with many natural gas fields, there is also the additional advantage that such nitrogen, which is unaffected by reforming, has a tendency to leave the molecular sieve unit with the hydrogen. Any nitrogen so doing thereby reduces the size of any nitrogen-producing unit needed in the production of ammonia.

At least some of the synthesis gas produced in the reforming step is thus fed to a molecular sieve containing separation unit, preferably that utilizing pressure swing adsorption (PSA) techniques, although thermal swing adsorption (TSA) techniques, or a combination of the two, may be used. Three streams are produced by the reforming and gas separation steps:-

A.   a stream containing hydrogen, a low (relative to B below) concentration of carbon monoxide and the balance of the other gases from the reforming step;

B.   a stream rich in carbon monoxide; and

C.    a stream containing substantially pure hydrogen.

Although a single column PSA unit can be used to produce all three streams, it is preferred to use at least two columns, or more generally two separation stages, in series.  In the first stage nearly all, e.g. about 95% by volume, of any carbon dioxide present in the synthesis gas is split off, taking with it some hydrogen, a relatively little carbon monoxide and most of the other gases, usually except nitrogen, from the reforming step.  This first split yields Stream A, which would conventionally be regarded in the PSA art as the "waste" stream.

The main stream which is left is then fed to the second PSA stage where carbon monoxide is split off from substantially pure hydrogen.  The purity of the carbon monoxide achieved in this separation stage is at least 70 mol %, whilst that of the hydrogen is generally greater than 99 mol %, the latter depending on the amount of nitrogen initially present in the natural gas feedstock, since nitrogen tends to leave with hydrogen.

In a preferred flowsheet arrangement, all of these three streams are produced by one main PSA unit, but alternative arrangements are possible as will be described hereinbelow.  In the preferred embodiment, each of these three streams is utilized as a feedstock for a separate synthesis unit. At least some of, and preferably all of, Stream A, either alone or in combination with some of the synthesis gas which was not fed to the molecular sieve unit and/or with some of Stream C is fed, generally after compression, to a methanol-synthesis unit, preferably a conventional loop.  By this means, unusually and with advantage, this unit may be arranged such that the ratio of carbon oxides to hydrogen is the optimum required for a minimum inter-unit transfer price of the methanol so formed. This ratio could well differ from the standard ratios for simple natural gas-to-methanol plants, which are normally fed with all of the synthesis gas produced. The crude methanol from the methanol unit may be distilled if required, but such distillation need only remove the majority of the water, and other non-hydrocarbon and non-carbohydrate chemicals which may be present.

As an alternative to a conventional methanol synthesis loop, a "mixed alcohol" synthesis process, such as Snam Progetti's MAS process, could be used. Such mixed alcohol processes produce a substantial amount of methanol along with higher alcohols. Although such a mixed alcohol stream could be fed direct into the "acetate" production unit, it may be that the percentage of higher alcohols present is high for the specification of the final alcohol product of the overall process. For example, when used to produce ethanol for petrol, the process may give ethanol containing so much higher alcohol that the octane number of the final blended petrol is unacceptably low. For such cases, it is desirable to separate, e.g. by distillation, the majority of the methanol from the crude mixed alcohol product before it is fed into the acetate unit. Such separation need not be such as to yield methanol of high purity, and could be included as part of the crude methanol distillation referred to above. The separated higher alcohols can either be processed and sold as separate products or be combined with the ultimate alcohol product of overall process to yield a mixed alcohol product, e.g. for blending with petrol.

Stream B together with the product methanol is fed to a standard acetic acid and/or ethyl acetate and/or acetic anhydride production unit. It will be appreciated, however, that the distillation section of such a unit need only be arranged to remove some of any water and some of any inorganic compounds present, since there is no need for this unit to produce, e.g. acetic acid, of the quality usually sold commercially for e.g. vinyl acetate monomer production. This gives the acetate stream.

This acetate stream is then combined with some of Stream C and is fed to an ethanol-synthesis unit. If such a unit produces an aqueous product, which is the case with acetic acid, it may be dehydrated by distillation or by permeable membrane or molecular sieve techniques to form anhydrous ethanol suitable for automobile use. DE 3221077 A1 of BASF discloses a very suitable process for hydrogenating acetic acid to ethanol.

As an alternative to feeding Stream A to a methanol loop, some or all of it may be recycled back to the reforming step. This would be advantageous if, because of its composition, this stream had the effect of increasing the concentration of the carbon monoxide in the synthesis gas. The second option is, however, only possible if the methanol is produced by a different route, i.e. via a second reformer.

The purge streams from the methanol and acetate synthesis units may be utilized as follows. If acetic acid is produced, its synthesis unit gas purge stream may be recycled either to the reformer or to the molecular sieve unit. Alternatively the purge stream can be fed to a second, dedicated molecular sieve unit in order to remove and recover CO from the stream. The purge stream from the methanol unit may likewise be recycled or fed to a further, dedicated molecular sieve unit to recover hydrogen. However, care must be taken if recycle is considered to ensure that there is build up neither of unreacted natural gas (methane) nor of any inerts, e.g. nitrogen, contained in the original natural gas, if used.

Because of possible methane build-up part of either or both of the above two recycle streams generally has to be recycled to the reformer or flared. If the natural gas contains inerts, e.g. nitrogen, a portion thereof is generally flared or used as a fuel in, e.g. a gas turbine or boiler.

Where a molecular sieve unit is used in the present process, other than as the main gas separation unit downstream of a single reforming stage, it need not produce three streams, but can in accordance with conventional practice just produce two streams. Thus, for example, if a purge gas PSA unit is designed to recover hydrogen, then there is no need to take off any "first stage" stream. On a flowsheet this can be shown diagramatically by reuniting the apparently-split streams immediately on leaving the separation unit.

When sufficient steam is fed with the natural gas to the reformer, be it of tubular or partial oxidation type, an excess of hydrogen over and above that required for the production of methanol and ethanol can be produced. For tubular steam reforming, steam-to-carbon ratios in the feed to the reformer should be at least 0.5:1, and preferably greater than 1.3:1. For partial oxidation reforming they can be somewhat lower. Although it can be burnt or used elsewhere, this hydrogen, after passage through the molecular sieve unit, is in a very pure form. Accordingly this invention preferably utilises the remainder of this pure Stream C to produce ammonia. The required nitrogen can be produced cryogenically, e.g. from air, at high purity, i.e. containing less than a few hundred ppm of impurities, mainly argon.

The criterion for deciding how to optimise the hydrogen and/or nitrogen stream purities is that the combined

**0167300**

hydrogen and nitrogen stream fed to the ammonia synthesis loop should contain less than about 5 ppm of oxygen in total of elemental and compound forms. Oxygen-containing compounds in the hydrogen would be mainly carbon monoxide, whilst elemental oxygen would be the impurity in the nitrogen stream. If the combined hydrogen and nitrogen streams contained less than about 5 ppm elemental plus compound oxygen, then the combined stream can be fed directly into the ammonia synthesis loop, otherwise it should be purified beforehand.

In order to deal with any trace excess of oxygen, the oxygen can be reacted with the hydrogen in the combined stream, and the resulting water can be adsorbed on, e.g. a molecular sieve, prior to entry into the ammonia synthesis loop. Any trace excess of carbon monoxide may likewise be reacted with hydrogen to form trace methane - typically at the few hundred ppm level - which is inert in the ammonia loop. These two reactions can be carried out simultaneously over a methanation-type catalyst. In addition, since these reactions are exothemic, it could be ensured that an amount of oxygen in the nitrogen was deliberately present so that any desired temperature rise took place so as to allow a feed-effluent heat exchanger to be used. This would also negate the need to utilise a nitrogen stream which was very pure with respect to oxygen. Inasmuch as the two reactants are very pure, when compared with normal ammonia plant synthesis gas, there would be a much slower build up of inert substances, mainly argon, in the ammonia synthesis loop, and a number of advantages would result. For example, not only would the partial pressure of the reactants be higher for any given synthesis pressure, but also the purge flow from the loop would be much reduced, which latter has further advantages well known to those versed in the art.

Because the feedstreams to the ammonia loop are independent in origin, the $H_2/N_2$ ratio in the loop need not be restricted, but can be easily adjusted optimally to take account of favourable reaction rates and catalyst development. In particular, this ratio can be independent of the composition of the make-up gas, in that if the loop is initially filled with, say, excess nitrogen this excess nitrogen can be kept in the loop and, in combination with certain catalysts, can promote a faster rate of reaction or a greater degree of reaction. There will be some loss of reactants because these will dissolve in the liquid ammonia product, but these can be flashed off and recycled to the loop in accordance with normal practice.

An alternative method of producing the ammonia would be to compress air and, preferably using steam as a moderator, to burn the oxygen directly out of the air stream using some of the excess hydrogen. Cooling of the resulting hot gases raises steam, which could be used in other parts of the process. After drying, a synthesis gas is left which is suitable as make-up gas for the ammonia synthesis loop, again generally after trace carbon oxides have been methanated out prior to drying. (The carbon here arises from carbon dioxide in the air.) Because some of the hydrogen is burnt, there is a reduction in the amount of ammonia which can be produced, but here again, because Stream C may easily be made very pure, it is still very economic to convert it to ammonia.

Inasmuch as the overall process is more economic at a large scale, it is advantageous to utilise two or more tubular steam reformers. Here there are advantages in designing different outlet conditions for these reformers. The reformer whose effluent will all or substantially all be fed to the main carbon monoxide molecular sieve unit can be run with a low steam:carbon

-12-                                      0167300

ratio, a low pressure and a high outlet temperature,
whereas the reformer whose effluent is partially or wholly
fed to the methanol-synthesis loop can have a higher
outlet pressure.

As is normal practice, compressors are needed to raise the
pressure of various streams throughout the process.
Typically, the low pressure tubular reformer would be
operated in the range of 3 to 30 ATM abs (0.3 to 3 MPa),
but preferably in the range of 6 to 15 ATM abs (0.6 to 1.5
MPa). That feeding the methanol loop would be operated in
the range of 10 to 40 ATM abs (0.1 to 4 MPa), but
preferably in the range of 15 to 30 ATM abs (1.5 to 3 MPa).

Waste water streams from the methanol or ethanol
distillation units may be utilized, possibly after pH
adjustment, in a natural gas saturator thereby recycling
residual carbon compounds back to the reformer, minimising
liquid effluent and indeed re-using water in areas where
it is in short supply. Unfortunately, water is often
scarce in areas where large supplies of natural gas are to
be found.

As will be readily appreciated, it is not necessary for
the overall process to be self-sufficient in methanol.
For economic reasons, it may be desirable to produce only
a portion of the total quantity of methanol needed and to
buy in the remainder.

Furthermore, it may be preferable to supplement the main
molecular sieve unit by a gas-permeable membrane unit,
such as a "Prism" unit. When positioned upstream thereof
such a Prism unit can be used to remove some hydrogen and
some carbon dioxide from the feed stream to the main
carbon monoxide molecular sieve unit, thereby reducing the
removal duty, and hence size, of the downstream unit, particularly because the molecular sieve unit then need not handle the major $CO/CO_2$ separation. Preferably the carbon dioxide separated by permeation is combined with Stream A from the downstream molecular sieve unit, adjusting pressures as necessary. In certain cases, for example when the feedstock contains significant amounts of nitrogen, a Prism unit can with advantage be positioned downstream of the molecular sieve unit.

A further option is to use two molecular sieve units, rather than one, optionally with the membrane unit positioned between the two. Whilst the downstream unit would operate as before producing the three defined streams, the upstream molecular sieve unit can be arranged to divide the main gas stream into just two streams by splitting off a large proportion of the hydrogen therein. This pure hydrogen stream can then be combined with Stream C of the downstream molecular sieve unit, or fed direct to the methanol synthesis unit. Re-compression of the gas stream between the various gas separation units may be necessary.

Yet another option is to replace the or both main molecular sieve units by the combination of a physical or chemical carbon monoxide selective absorption unit to yield Stream B, followed by one molecular sieve unit to produce just Streams A and C. Such absorption units are well known in the art and include those using the "Cosorb" process, as well as copper liquor wash and copper chloride/solvent processes. Again an upstream gas-permeable membrane unit can be used to separate off some of the hydrogen and carbon dioxide before carbon monoxide removal takes place, or a by-pass to Stream A can be used.

The process of the present invention can also handle without substantial alteration natural gas which contains

some $CO_2$. However, if the feedstock natural gas contains a significant proportion of carbon dioxide, then an additional process step can be introduced to "shift" some of the carbon monoxide in the stream from the natural gas reformer in order to obtain sufficient hydrogen to ·hydrogenate the acetate stream to ethanol. In that case, however, the use of the molecular sieve unit to give a CO stream and a hydrogen stream is advantageous in that, in order to obtain the CO, an extremely high hydrogen recovery can be effected relative to normal hydrogen-producing molecular sieve units.

This invention generally eliminates the need separately to remove carbon dioxide from process streams. Dedicated carbon dioxide removal units are relatively expensive to construct and, having a number of rotating equipment items handling liquids, require significant maintenance. Furthermore their reliability is usually less than that of molecular sieve units. These factors are important where plant is operating in remote geographical areas.

Although the present invention has been described in terms of the production of ethanol, it will be appreciated by those skilled in the art that higher alcohols, e.g. propanol, can similarly be formed by using the product ethanol in place of the original methanol feed into the "acetate" - or generally "alkanoate" - synthesis unit. Higher aliphatic alcohols can thereby be formed, with n in the formula of the intermediate compound taking the value 1, 2 etc. In addition a mixture of alcohols can be produced either by recycling some of the product alcohol to step (c) to act as an intermediate alcohol in the production of a higher alcohol, or by using a "mixed alcohol" synthesis process as described earlier instead of a synthesis unit producing essentially methanol. Reaction conditions and residence times can be controlled to limit the formation of by-products, e.g. unwanted alcohols of undesirably high molecular weight, and to form the desired alcohol blend.

Various embodiments of the present invention will now be described by way of example, with reference to the accompanying drawings, in which:-

Figure 1 is a schematic flow-scheme for the overall process of the invention;

Figures 2 and 3 illustrate schematically the main options for step (a) of Figure 1;

Figures 4, 5, 6, 7 and 8 illustrate schematically the main options for the gas separation step of Figures 2 and 3;

Figures 9 and 10 illustrate schematically the main options for the molecular sieve gas separation step of Figures 4, 5, 6, 7 and 8;

Figures 11, 12 and 13 illustrate schematically the main options for the reforming processes of Figures 2 and 3;

Figures 14 and 15 illustrate schematically the main options for the reformer used in the reforming processes of Figures 11, 12 and 13; and

Figure 16 is a flowsheet for the overall production of ethanol and ammonia from methane, in which two steam reformers are used with two purge gas PSA units in addition to the main synthesis gas PSA unit.

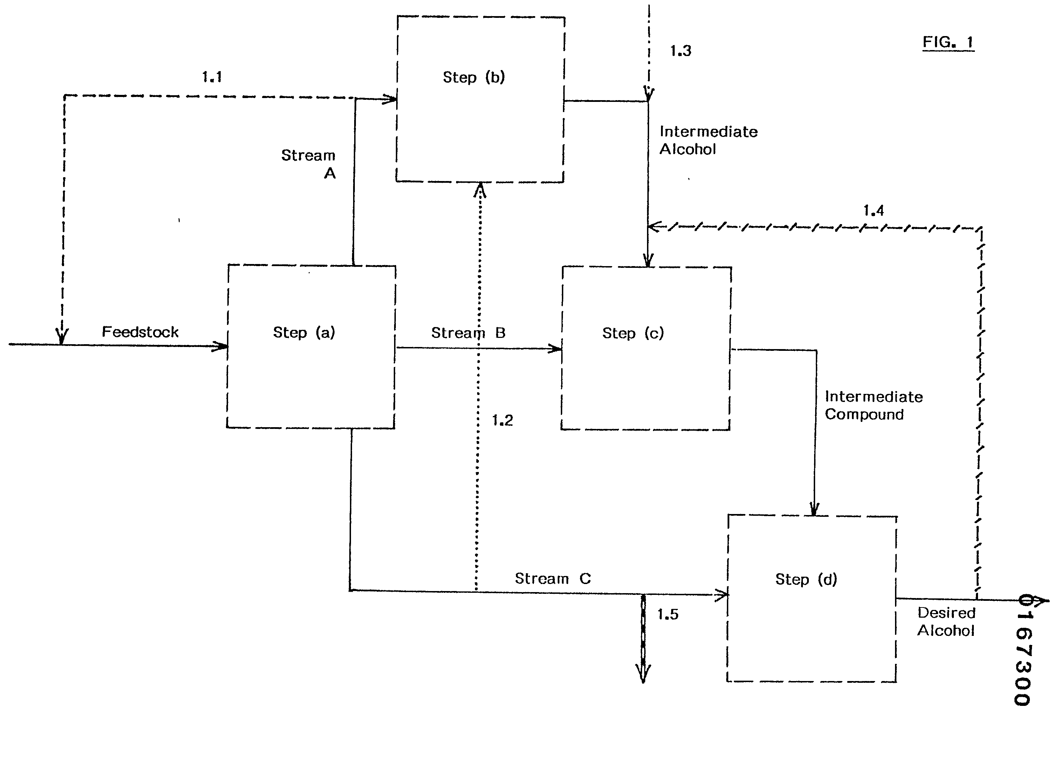

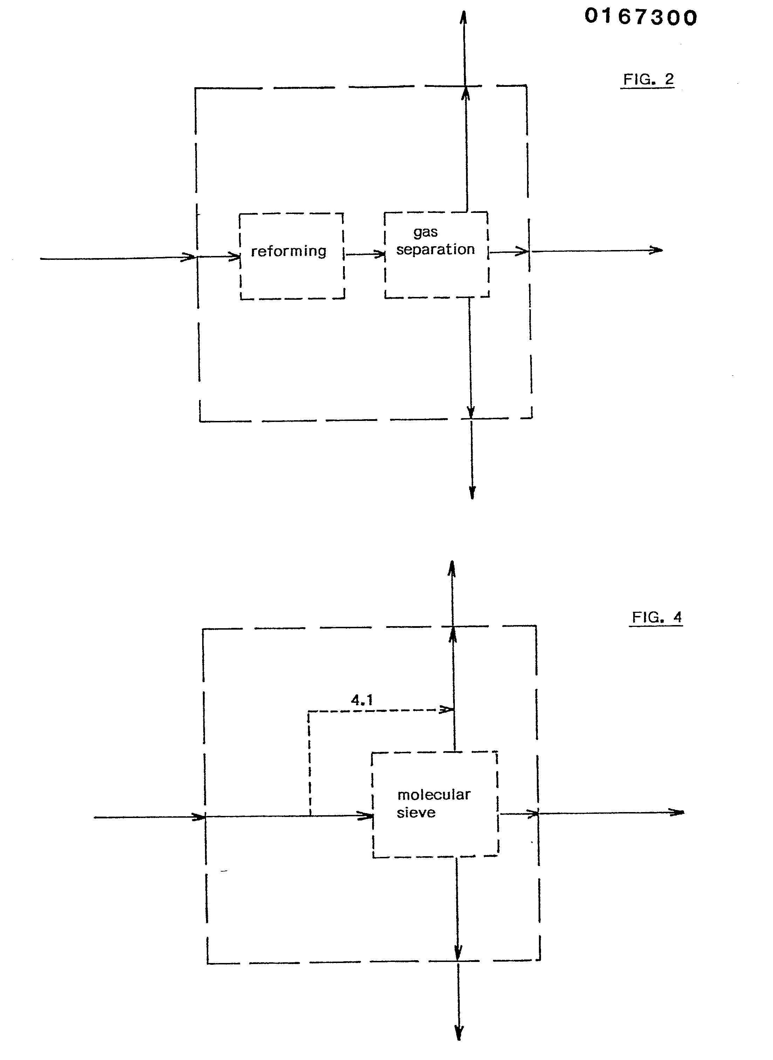

In Figures 1 to 15, the solid flowlines represent the fixed flowpaths, whilst the dotted flowlines represent the main alternative options available for the illustrated process. Generally the different options of one Figure can be combined together as desired. Also any of the various options illustrated in the different Figures can be combined freely as may be required for particular process conditions.

In Figure 1, the carbonaceous feedstock is reformed and separated in step (a) to yield Streams A, B and C, which in turn are reacted together in steps (b), (c) and (d) to produce the desired aliphatic alcohol. The main options which are illustrated in Fig. 1 are:-

1.1 some recycle of Stream A to the feed to step (a),

1.2 the reaction of some of Stream C in step (b),

1.3 the buying-in of outside intermediate aliphatic alcohol where that produced by step (a) is less than that needed for step (c),

1.4 the recycle of some of the desired alcohol to supplement or replace the intermediate alcohol being fed to step (c), and

1.5 the concurrent production of ammonia from Stream C excess to the requirements of step (d).

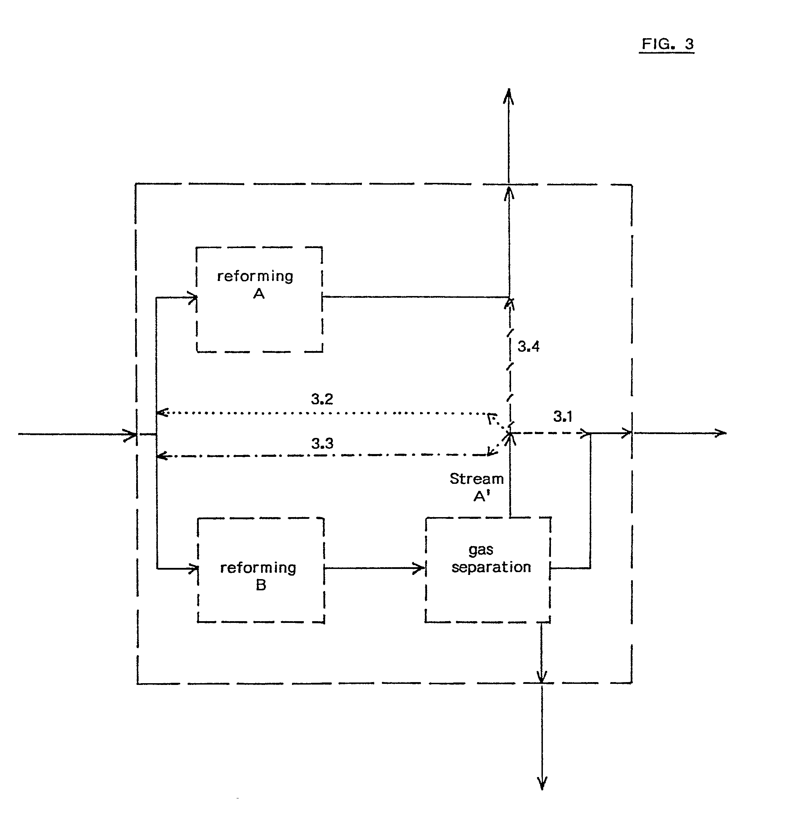

Figures 2 and 3 illustrate the main options for step (a) illustrated in Fig. 1. For Fig. 2, the single main option is the use of a single reforming step followed by a gas separation step yielding Streams A, B and C.

In Figure 3 the feedstock is divided into two streams, each of which is reformed, in reforming processes A and B respectively, but only one of which - that from reforming process B - is subjected to a gas separation step.  Here the main options concern the extent of the gas separation and the destination of any third stream produced thereby. The main options are:-

    3.1  gas separation into only two Streams B and C,

    Where there is gas separation into Streams B, C and a third Stream A' containing hydrogen, a low (relative to Stream B) concentration of carbon monoxide and a balance of the other gases in the synthesis gas stream being separated, the following main options apply:-

    3.2  recycle of Stream A' to the feed to reforming process A,

    3.3  recycle of Stream A' to the feed to reforming process B, and

    3.4  the combining of Stream A' with the effluent from reforming process A to produce Stream A.

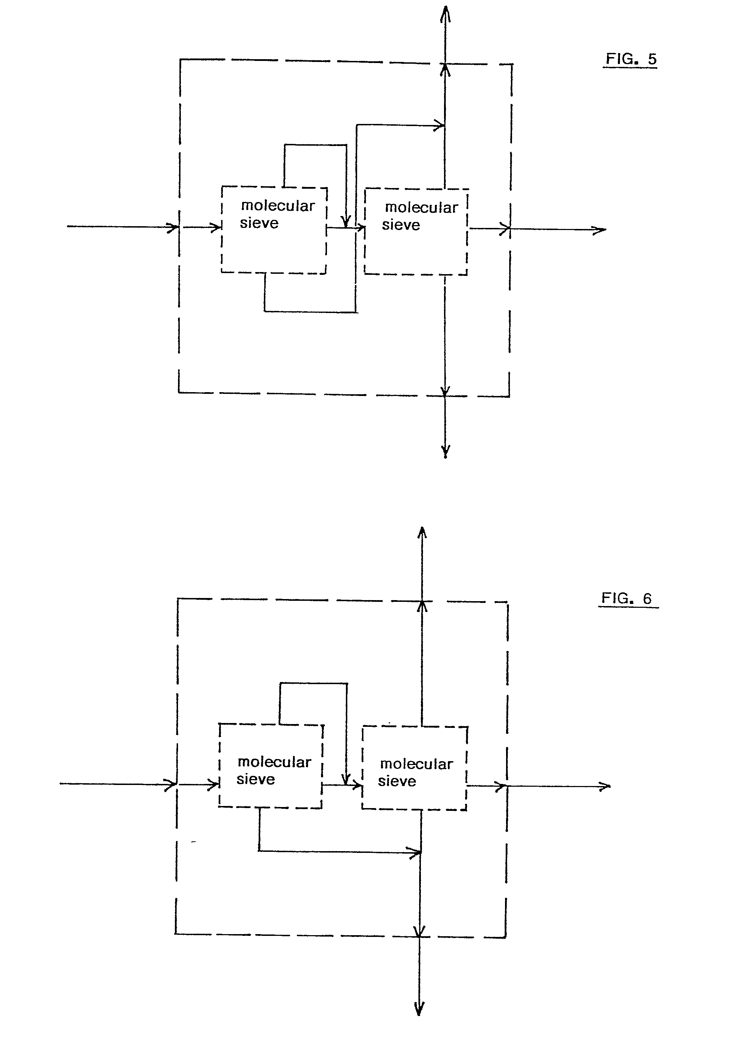

Figure 4 shows the use of a molecular sieve as the sole gas separation device, with its main option being:-

    4.1  some by-pass of the feed to the molecular sieve direct into Stream A.

In Figures 5 and 6 two molecular sieves are used in series with the first sieve being used to produce just two streams, rather than the three streams formed by the downstream sieve.  The substantially pure hydrogen which is split off by the first sieve from the synthesis gas stream is combined with Stream A or A' in Figure 5 and with Stream C in Figure 6.

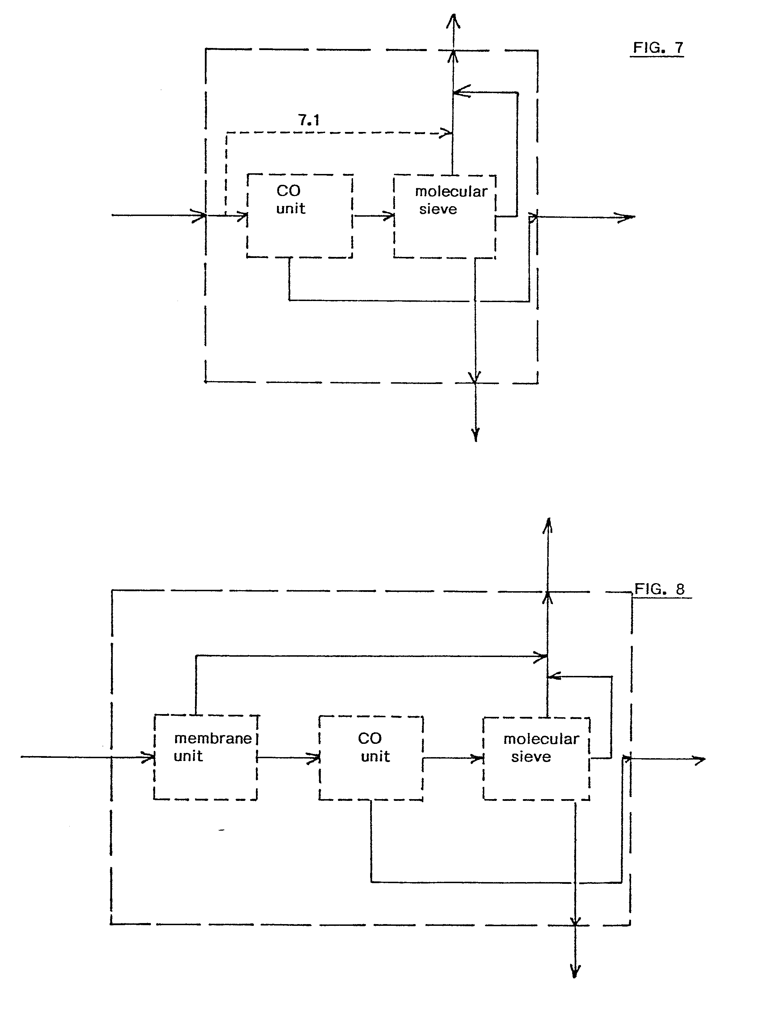

In Figures 7 and 8, a carbon monoxide-selective absorption/desorption system is used in combination with a molecular sieve to split off from the synthesis gas stream the CO-rich stream B. The downstream molecular sieve then separates Stream A or A' from the substantially pure hydrogen stream, Stream C. For the arrangement of Figure 7, the single main option is:-

7.1 some by-pass of the synthesis gas direct into Stream A without being separated.

In Figure 8, a stream is similarly taken off upstream of the main separation units and combined with Stream A, but here the stream is split off by a gas membrane unit. The by-passing stream in this case is essentially a mixture of hydrogen and carbon dioxide.

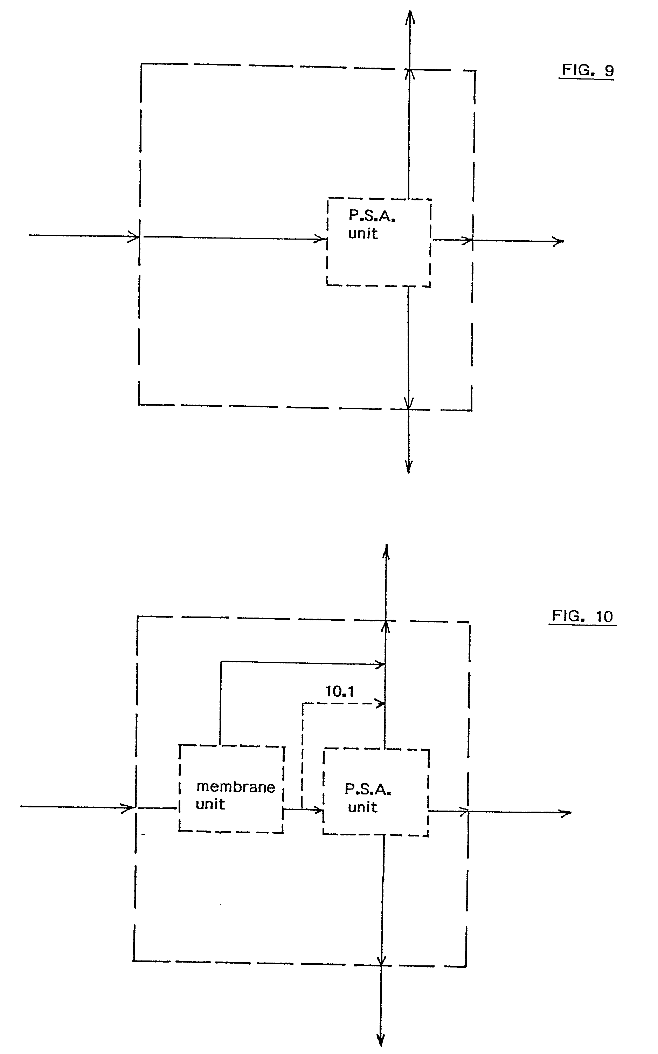

Figures 9 and 10 illustrate the main options for the molecular sieves of Figures 4 to 8. Figure 9 shows a pressure swing adsorption (PSA) unit alone, whilst Figure 10 shows the combination of a PSA unit with a gas membrane, the membrane again functioning as in Figure 8. The main option for Figure 10 is:-

10.1 some by-pass of the PSA feedstream around the PSA unit direct into Stream A or A'.

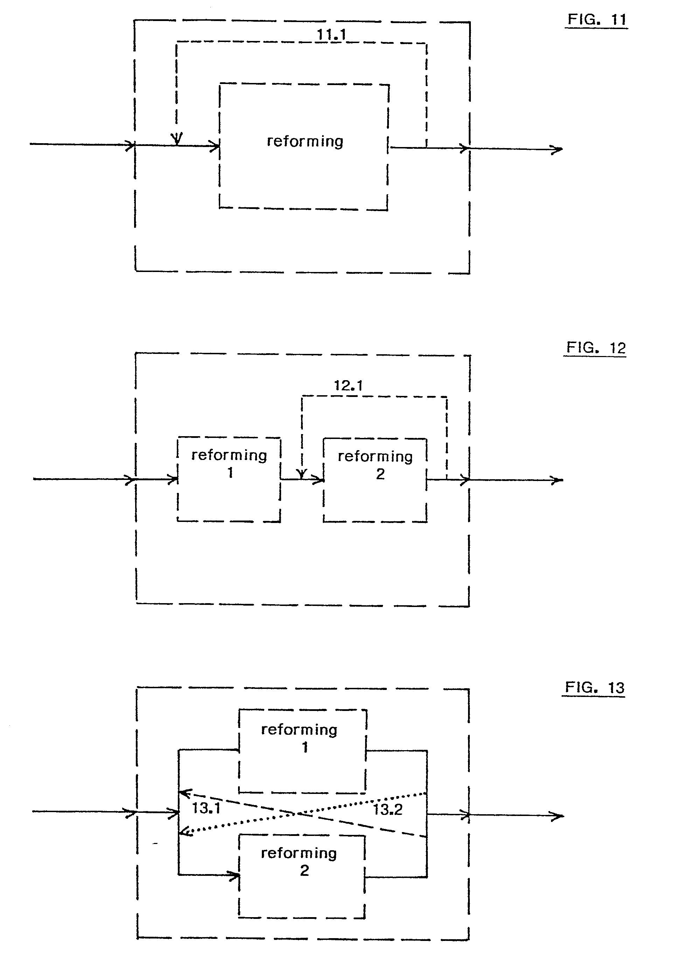

Figures 11, 12 and 13 illustrate the different options for the reforming processes used in Figures 2 and 3. Figure 11 shows the use of a single reforming step, whilst Figures 12 and 13 show the use of two reforming processes in series and in parallel, respectively. The main options are all based on some recycle of the reformed gases back to the reforming process.

In 11.1 the recycle is around the whole reforming process whilst in 12.1, 13.1 and 13.2 the recycle is to an intermediate point in the reforming process. Specifically in:-

12.1 the recycle from the second, downstream reforming process is returned only to the second reforming process,

13.1 the recycle from the second parallel reforming process is returned to the first parallel reforming process,

13.2 the recycle from the first parallel reforming process is returned to the second parallel reforming process.

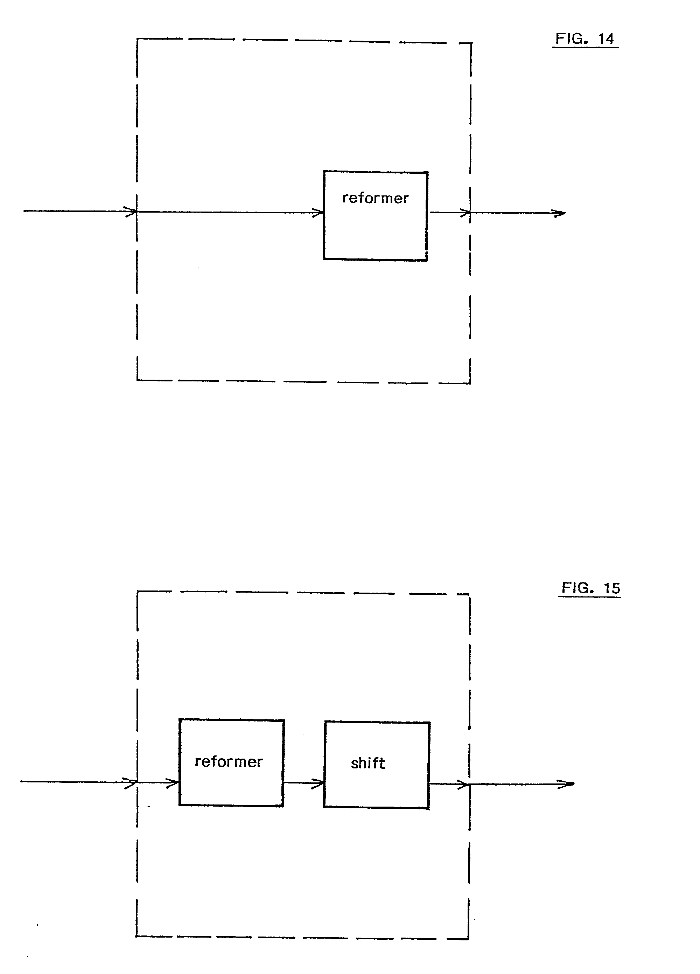

Figures 14 and 15 show the two main options for the reforming process of Figures 11 to 13, namely, respectively, a reformer on its own and when combined with a $CO/CO_2$ shift unit. For each reformer a tubular steam reformer or a partial oxidation reformer can be used.

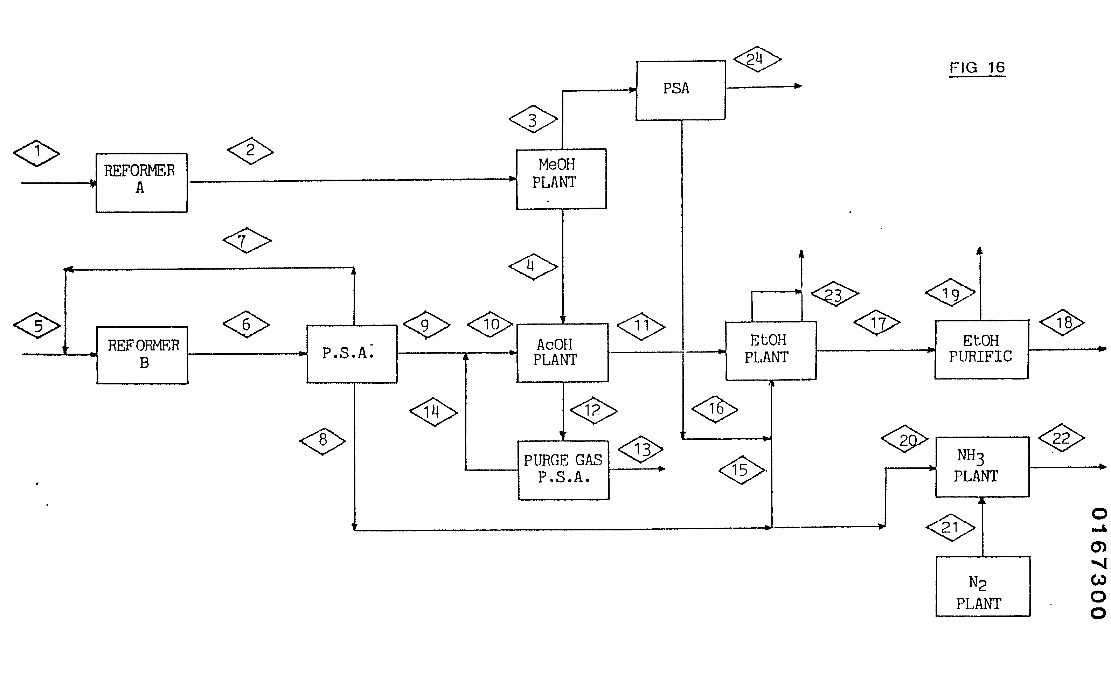

Figure 16 illustrates the general overall flowsheet for a preferred process of the invention for producing ethanol concurrently with ammonia from a natural gas feedstock.

The natural gas feedstock consisting of methane with a trace amount of nitrogen is split into two streams 1 and 5 and is fed respectively into two tubular steam reformers, Reformer A and Reformer B. The synthesis gas effluent 2 from Reformer A is fed to a methanol (Me OH) plant, whilst that 6 from Reformer B is separated by a main pressure swing adsorption (PSA) unit into streams 7, 8 and 9. Stream 8 consists of substantially pure hydrogen and is divided into streams 15 and 20 to be fed, respectively, into an ethanol (Et OH) plant and an ammonia ($NH_3$) plant. Stream 9 consists substantially of carbon monoxide and constitutes the major part of the feed 10 for the acetic acid (Ac OH) plant, whilst stream 7 - the "waste" stream - is recycled to Reformer B.

In the Me OH plant a methanol stream 4 is produced which is fed direct into the Ac OH plant. The residual gases 3 from the Me OH plant are separated by an Me OH purge gas PSA unit in order to recover substantially pure hydrogen 16 which is combined with stream 15 and supplied to the Et OH plant. The rejected gases 24 from this PSA unit are used as a fuel for the process, because of their good calorific value.

Streams 4 and 10 are reacted together in the Ac OH plant to yield acetic acid 11 which is then fed into the Et OH plant to be converted by the hydrogen of streams 15 and 16 into the raw ethanol product 17. The purge gases 12 from the Ac OH plant are separated by an Ac OH purge gas PSA unit in order to recover any unreacted carbon monoxide which is recycled 14 back to the Ac OH plant. The rejected gases 13 from this PSA unit are used as fuel.

Useful heat can also be recovered from the effluent stream 23 rejected by the Et OH plant.

The raw ethanol 17 is purified and dehydrated by the Et OH purification unit to yield an ethanol stream 18 of a purity and water content suitable for use in automobiles. The water-containing waste from this purification unit is generally used in a natural gas saturator (not shown) in order to recycle both water and residual carbon-containing compounds back to the main process.

Meanwhile a nitrogen ($N_2$) plant has been producing substantially pure nitrogen 21 which is combined with the hydrogen stream 20 in the $NH_3$ plant to yield ammonia 22.

0167300

A small gas purge from the $NH_3$ plant may be necessary, dependent on the amount of argon designed for in the nitrogen plant.

A mass balance for the flowsheet of Figure 16 is set out in Table 1 below. The figures given are all expressed in kilogramme-moles per hour, and the basis for the flowsheet is 2500 tonne/day of product ethanol in stream 18. Where the word "variable" appears in the table, this means that the precise figure is not fixed but can vary or can be varied as desired in accordance with prevailing cost and energy considerations. For any given situation the precise figure can easily be evaluated by one skilled in the art.

## TABLE 1

| STREAM | | 1 | 2 | 3 | 4 | 5 | 6 | 7 | 8 |
|---|---|---|---|---|---|---|---|---|---|
| $CH_4$ | M.Wt. 16 | 2890.5 | | 326.3 | | 2698.1 | 160.0 | 80.0 | |
| CO | 28 | | | 82.2 | | | 2724.8 | 272.0 | 0.8 |
| $CO_2$ | 44 | | | 76.4 | | | 800.7 | 635.4 | |
| $H_2$ | 2 | | | 2832.1 | | | 8154.2 | 135.8 | 7991.1 |
| $H_2O$ | 18 | | | Variable | 4.2 | | | | |
| MeOH | 32 | | | 14.3 | 2361.7 | | | | |
| AcOH | 60 | | | | | | | | |
| EtOH | 46 | | | | | | | | |
| $NH_3$ | 17 | | | | | | | | |
| $N_2$ | 14 | 12.6 | | 12.5 | | | | | |
| | | | | | | | | | |
| TOTAL | | 2903.1 | Variable | Variable | 2365.9 | 2698.1 | 11839.7 | 1123.2 | 7991.9 |
| | M.T.D. | | | | 1815.6 | | | | |

## TABLE 1 cont.

| STREAM | | ◇9 | ◇10 | ◇11 | ◇12 | ◇13 | ◇14 | ◇15 | ◇16 |
|---|---|---|---|---|---|---|---|---|---|
| | M.Wt. | | | | | | | | |
| $CH_4$ | 16 | 80.0 | 100.8 | | 100.8 | 80.0 | 20.8 | | |
| CO | 28 | 2451.6 | 2867.0 | | 461.6 | 46.2 | 415.4 | 0.3 | 0.2 |
| $CO_2$ | 44 | 165.3 | 186.1 | | 257.8 | 237.2 | 20.8 | | |
| $H_2$ | 2 | 27.3 | 31.9 | | 103.6 | 99.0 | 4.6 | 2553.7 | 2265.7 |
| $H_2O$ | 18 | | | Variable | | | | | |
| MeOH | 32 | | | | | | | | |
| AcOH | 60 | | | 2333.7 | | | | | |
| EtOH | 46 | | | | | | | | |
| $NH_3$ | 17 | | | | | | | | |
| $N_2$ | 14 | | | | | | | | |
| | | | | | | | | | |
| TOTAL | | 2724.2 | 3185.8 | Variable | 923.8 | 462.4 | 461.6 | 2554.0 | 2265.9 |
| | M.T.D. | | | | | | | | |

# TABLE 1 cont.

| STREAM | | 17 | 18 | 19 | 20 | 21 | 22 | 23 | 24 |
|---|---|---|---|---|---|---|---|---|---|
| CH$_4$ | M.Wt. 16 | | | Variable | | | | 23.4 | 326.3 |
| CO | 28 | | | | 0.6 | | | | 82.0 |
| CO$_2$ | 44 | | | | | | | | 76.4 |
| H$_2$ | 2 | | | | 5437.4 | | | 128.7 | 566.4 |
| H$_2$O | 18 | Variable | | | | | | | |
| MeOH | 32 | | | | | | | | 14.3 |
| AcOH | 60 | | | | | | | | |
| EtOH | 46 | 2275.8 | 2264.5 | | | | | | |
| NH$_3$ | 17 | | | | | | 3606.8 | | |
| N$_2$ | 14 | | | | | Variable | | | 12.5 |
| | | | | | | | | | |
| TOTAL | | Variable | 2264.5 | Variable | 5438.0 | Variable | 3606.8 | 152.1 | 1077.9 |
| | M.T.D. | | 2500 | | | | 1471.6 | | |

CLAIMS:

1.   A process for the production of an aliphatic alcohol having at least two carbon atoms from a carbonaceous feedstock, which process comprises:-

a)  reforming the feedstock to yield synthesis gas containing carbon monoxide, optionally together with carbon dioxide, and hydrogen;

b)  reacting together some of the carbon oxide(s) from step (a) and some of the hydrogen from step (a) to form an intermediate aliphatic alcohol having at least one carbon atom;

c)  reacting together carbon monoxide from step (a) and the intermediate alcohol from step (b) to form an intermediate compound containing the group $CH_3(CH_2)n\ C(O)-$ wherein n is 0 or a positive integer, and

(d)  reacting the intermediate compound from step (c) with more of the hydrogen from step (a) to form the desired aliphatic alcohol;

wherein step (a) includes the separation of at least some of the synthesis gas whereby step (a) produces:-

A.  a stream containing hydrogen, a low (relative to Stream B below) concentration of carbon monoxide and a balance of the other gases in the synthesis gas;

B.  a stream rich in carbon monoxide; and

C.  a stream containing substantially pure hydrogen,

wherein at least some of Stream A is reacted in step (b), at least some of Stream B is reacted in step (c), and at least some of Stream C is reacted in step (d).

2. A process as claimed in claim 1 wherein a portion of Stream A is recycled to the reforming process of step (a).

3. A process as claimed in claim 1 or claim 2 wherein a portion of Stream C is reacted in step (b).

4. A process as claimed in any one of the preceding claims wherein additional intermediate alcohol from a source other than step (b) is reacted in step (c).

5. A process as claimed in any one of the preceding claims including further steps (c) and (d) in which a first desired alcohol is used as a further intermediate alcohol to form a second desired alcohol having one more carbon atom than the first desired alcohol.

6. A process as claimed in any one of the preceding claims wherein some desired alcohol product is recycled to step (c) in order to produce after step (d) a mixture of aliphatic alcohols.

7. A process as claimed in any one of the preceding claims wherein a portion of Stream C is used to produce ammonia.

8. A process as claimed in any one of the preceding claims wherein in step (a) the feedstock is reformed to a single synthesis gas stream which is separated into Streams A, B and C.

9. A process as claimed in any one of claims 1 to 7 wherein in step (a) the feedstock is reformed to two synthesis gas streams, one of which constitutes Stream A and the other of which is separated into Streams B and C.

10. A process as claimed in claim 9 wherein the said other synthesis gas stream is separated into Streams A', B and C, wherein Stream A' contains hydrogen, a low (relative to Stream B) concentration of carbon monoxide and a balance of the other gases in the said other synthesis gas stream.

11. A process as claimed in claim 10 wherein Stream A' is recycled to the reforming step of step (a).

12. A process as claimed in claim 10 wherein Stream A' is combined with Stream A.

13. A process as claimed in any one of the preceding claims wherein the gas separation step includes the use of a molecular sieve.

14. A process as claimed in claim 13 wherein a portion of the gas stream fed to the molecular sieve by-passes the molecular sieve.

15. A process as claimed in claim 14 wherein the by-pass portion is combined with Stream A or Stream A'.

16. A process as claimed in claim 14 or claim 15 wherein the by-pass portion is separated from the feedstream by a molecular sieve.

17. A process as claimed in claim 16 wherein the by-pass portion consists of substantially pure hydrogen, at least some of which is combined with Stream C.

18. A process as claimed in claim 14 wherein the by-pass portion consists substantially of carbon monoxide and is separated from the feedstream by a carbon monoxide-selective absorption/desorption process.

19. A process as claimed in claim 18 wherein the carbon monoxide-containing by-pass portion constitutes Stream B, whilst the molecular sieve separates Stream C from Streams A or A'.

20. A process as claimed in claim 19 wherein a portion of the gas stream fed to the carbon monoxide-selective absorption/desorption process by-passes the said absorption/desorption process.

21. A process as claimed in claim 20 wherein the carbon monoxide by-pass portion is separated from the absorption/desorption process feedstream by a gas membrane.

22. A process as claimed in claim 21 wherein the carbon monoxide by-pass portion is combined with Stream A or Stream A'.

23. A process as claimed in any one of claims 13 to 22 wherein the molecular sieve operates within a pressure swing adsorption (PSA) gas separation unit.

24. A process as claimed in claim 23 wherein a portion of the gas stream fed to the PSA unit by-passes the PSA unit.

25. A process as claimed in claim 24 wherein the PSA by-pass portion is separated from the PSA unit feedstream by a gas membrane.

26. A process as claimed in claim 25 wherein the PSA by-pass portion is combined with Stream A or Stream A'.

27. A process as claimed in any one of the preceding claims the reforming step of step (a) includes recycling a portion of the reformed gases back to the reforming step.

28. A process as claimed in any one of the preceding claims wherein the reforming step of step (a) is carried out in two stages.

29. A process as claimed in claim 28 wherein the two reformer stages are in series.

30. A process as claimed in claim 28 wherein the two reformer stages are in parallel.

31. A process as claimed in any one of the preceding claims wherein the reforming step of step (a) includes a $CO/CO_2$ shift process.

32. A process as claimed in any one of the preceding claims wherein the reforming step of step (a) utilizes steam reforming.

33. A process as claimed in any one of claims 1 to 31 wherein the reforming step of step (a) utilizes partial oxidation reforming.

34. A process as claimed in any one of the preceding claims wherein the reforming conditions of step (a) are such that substantially pure hydrogen is produced excess to the requirements of steps (b) and (d).

35. A process as claimed in claim 34 wherein the excess hydrogen is reacted with nitrogen to produce ammonia.

36. A process as claimed in any one of the preceding claims wherein Stream B consists essentially of carbon monoxide, the impurities content being at least 8 mol %.

37. A process as claimed in any one of the preceding claims wherein the carbonaceous feedstock is a hydrocarbon.

38. A process as claimed in claim 37 wherein the hydrocarbon is natural gas.

39. A process as claimed in any one of the preceding claims wherein the intermediate aliphatic alcohol is methanol and the desired aliphatic alcohol is ethanol.

40. A process as claimed in claim 39 wherein the intermediate compound is acetic acid.

41. Ethanol when produced by a process as claimed in any one of the preceding claims.

42. A petrol extender/octane improver consisting of or containing ethanol as claimed in claim 41.

43. Petrol containing a petrol extender/octane improver as claimed in claim 42.

44. Ammonia when produced concurrently with the desired aliphatic alcohol in accordance with a process as claimed in any one of claims 1 to 40.

FIG. 1

0167300

0167300

FIG. 2

FIG. 4

FIG. 3

0167300

FIG. 5

FIG. 6

0167300

FIG. 7

7.1

| CO unit |

| molecular sieve |

FIG. 8

| membrane unit |

| CO unit |

| molecular sieve |

0167300

FIG. 9

P.S.A.
unit

FIG. 10

10.1

membrane
unit

P.S.A.
unit

0167300

FIG. 11

FIG. 12

FIG. 13

FIG. 14

reformer

FIG. 15

reformer    shift

0167300

FIG 16

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | WO-A-8 303 409 (DAVY McKEE) * Page 21, line 26 - page 25, line 20; description of figures 1-3 * | 1 | C 07 C 29/136 C 07 C 29/15 C 07 C 31/08 |
| A | EP-A-0 011 404 (ICI) * Page 9, line 1 - page 12, line 7; figure 1 * | 1 | |
| A | US-A-4 315 900 (SHINKISHI NOZAWA) * Column 3, line 44 - column 7, line 23; figurs 1,2 * | 1 | |
| A | FR-A-2 420 568 (TEXACO) * Page 21, line 2 - page 26, line 34; figures IA,IB * | 1 | TECHNICAL FIELDS SEARCHED (Int Cl 4) C 07 C 29/00 C 07 C 31/00 |

The present search report has been drawn up for all claims

| Place of search | Date of completion of the search | Examiner |
|---|---|---|
| THE HAGUE | 05-09-1985 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82